# EUROPEAN PATENT SPECIFICATION

(11) **EP 0 504 483 B1**
(45) Date of publication and mention of the grant of the patent: **09.08.1995**
(21) Application number: 91120965.8
(22) Date of filing: 06.12.1991
(51) Int. Cl.: A61K 38/23, A61K 9/02

(54) **Rectal compositions containing lyophilized calcitonin**
Arzneizusammensetzungen zur rektalen Verabreichung enthaltend gefriergetrocknetes Calcitonin
Compositions pour l'administration rectale à base de calcitonine lyophilisée

(30) Priority: 21.03.1991 IT RM910188
(43) Date of publication of application: 23.09.1992
(73) Proprietor: PULITZER ITALIANA S.r.l., 00156 Roma (IT)
(72) Inventor: Bertone, Evaristo, I-00156 Roma (IT)
(74) Representative: Bianchetti, Giuseppe

(56) References cited:
- EP-A- 0 399 781
- GB-A- 2 092 002
- US-A- 4 774 091
- DATABASE WPIL, accession no. 81-80457D [44], Derwent Publications Ltd, London, GB; & JP-A-56 118 013

## Description

The present invention relates to rectal pharmaceutical compositions containing lyophilized calcitonin.

Calcitonins are a well-known class of pharmacologically active polypeptides, whose therapeutic utility in Paget's disease, hypercalcemia and osteoporosis has been described.

Due to calcitonin peptidic nature, only the parenteral administration is actually used.

Recently, the rectal route proved to give, at acceptable dosages, a reliable adsorption, thus achieving a bioavailability comparable to that obtained with the normal parenteral administration.

On the other hand, rectal compositions, which give evident practical advantages, such as self-administration and lack of painful side effects, imply some technical difficulties:
1) the product must be homogeneous; the same being a dispersion of small amounts of active ingredient in a solid mass, homogeneity can be achieved only by the use of appropriate techniques.
2) the product must ensure a sufficient absorption.
3) the product must give no local irritations.
4) the product must be stable, possibly anhydrous.
5) the process for the preparation must be carried out at a low temperature during each step.

British Patent Specification No. 1354525 discloses suppositories containing calcitonin. The excipients are lactose, polyethylene glycol 400 and 4000, polysorbate 80, glycerol. Suppositories are buffered at pH 4.5 with lactic acid. No other particulars, neither about the composition, nor about the method for the preparation, are disclosed.

DE 3830245 discloses suppositories containing calcitonin which include taurocholic acid or the salts thereof as absorption promoters, a buffer, a hydrosoluble excipient, such as mannitol or lactose, and a mass consisting in semisynthetic glycerides. The suppository is anhydrous, therefore stable, however the process for the preparation thereof is quite difficult.

EP-A-308725 discloses rectal formulations consisting of gelatin capsules or microenemas wherein a hydrophilic liquid is dosed, which contains calcitonin, polyethylene glycols or polypropylene glycols, a buffer, a C₂-C₆ alcohol, a stabilizer. Said preparation is readily homogeneizable, calcitonin being dissolved in water and the excipients being hydrophilic. However, the stability is endangered by the presence of 5 to 15% water.

It has now been found that the use of lyophilized calcitonin in suppository galenic formulations involves the following advantages:
a) the active ingredient is supported with suitable excipients as to achieve a bulky, soft, uniform, easily fat-dispersable mass, with no need for sophisticated equipments;
b) the freeze-dried mass, being obtained from a solution, is certainly homogeneous;
c) the freeze-dried mass is characterized by a low residual humidity, lower than 0.3% w/w, which is obtained with a sufficiently high vacuum, so that to ensure a good stability;
d) the freeze-dried mass easily dissolves into aqueous liquids, thus promptly yielding even concentrated solutions; thereby promoting the absorption, and therefore the bioavailability;
e) each operation is performed at a temperature lower than 21°C, taking into consideration the thermolability of the active ingredient.

Accordingly, the invention relates to rectal pharmaceutical compositions in the form of suppositories which contain freeze-dried calcitonin in a suitable vehicle. Preferably, the compositions of the invention also contain an aqueous buffer which consists of tromethamine organic acid addition salts, which have surfactant properties and act as absorption promoters; a bactericide; optionally other additives, such as surfactants, dispersants, stabilizers, carriers.

Generally, the excipients which act as supports for calcitonin are those used in the freeze-drying technique: for instance, mannitol, glycine, lactose, etc., in amounts ranging between 1 and 15% w/v of the solution to be freeze-dried. In many cases, it is convenient to freeze-dry calcitonin and the support together with the buffer, the microbicide and the stabilizer. In this case, the suppository will simply consist of a freeze-dried mass homogeneously dispersed in the lipophilic or hydropilic mass.

Further, a bactericide, an absorption promoter, a buffer, a mass and a stabilizer can optionally be provided in the composition of the invention.

Microbicides can be selected from the group consisting of phenol compounds, such as p-hydroxybenzoates, in concentrations ranging from 0.1 to 0.2% w/v; quaternary ammonium compounds, such as benzalkonium chloride, in concentrations ranging from 0.005 to 0.3% w/v; mercurial compounds, such as merthiolate, in concentrations ranging from 0.001 to 0.01% w/v, etc. Said compounds are used in order to prevent microbial growth during the process for the preparation of the aqueous solution to be freeze-dried, and consequently to prevent the degradation by any bacterial proteases present.

Surfactants, particularly tromethamine or meglumine, are preferred as absorption promoters.

Tromethamine, which is known also as tris buffer, and meglumine are two amino bases with strongly hydrophylic properties; when properly salified with acids, said bases are effective buffers in the pH range between 3.5 and 8.

The characteristic promotion effect on rectal absorption of the above salts, is due both to the surfactant properties of the base, particularly tromethanine, and to the presence of the anion, which is suitably selected from the group consisting of acids which are known to promote the absorption of rectally-administered peptides, for instance, dicarboxylic, tricarboxylic, hydroxycarboxylic acids, such as malonic, maleic, fumaric, tartaric, citric, succinic, malic, glutamic, aspartic, glutaric, adipic, glucuronic acids.

Said salts are used in an amount between 1 and 10 mg per suppository.

For the preparation of suppositories, lipophilic masses, such as conventional hemisynthetic glyceride mixtures, or hydrophilic masses, such as polyethylene- or polypropylene glycol mixtures, can be used.

If lipophilic masses are used, 5 to 25% w/w vaseline oil is preferably added, in order to obtain a mixture having m.p. 33-33.5°C, as to carry out the preparation and partition steps at a temperature no higher than 33.5-34°C. If a hydrophilic mass is used, a C₃-C₆ alcohol, bearing one or two hydroxy groups, is conveniently used.

Albumin also can suitably be used to stabilize and especially to avoid or to decrease the well-known adsorption of active ingredient during the preparation of the aqueous solution to be freeze-dried. The word albumin, as herein used, comprises suitably purified albumin (fraction V) both human and bovine. Albumin is used in an amount ranging from 1 to 20 mg per suppository.

Also edetic acid and the salts thereof may advantageously be used as stabilizers in order to avoid the catalytic effects of traces of heavy metals which could be present in the product.

In order to avoid even the least interference with calcium, calcium disodium edetate is preferred among the various edetic acid salts, at doses comprised between 0.05 and 0.5 mg per suppository.

To the above cited formulations, other ingredients like surfactants (0.1-0.5% w/w), neutral or basic amino acids (0.1-5% w/w), acyl cholines and acyl carnitines (0.1-0.2% w/w), dispersants (0.01-1% w/w), such as lecithins, monostearates, acylsorbitans, carriers (0.05-1% w/w) like PVP and DMAC, which assist the absorption of rectally administered calcitonin, thereby enhancing its bioavailability, can also be added.

The word calcitonin, as used in the above stated compositions, comprises all natural calcitonins, as well as their derivatives and pharmacologically active analogues, which are obtained by modifying the parent compound.

The following examples further illustrate the invention.

### EXAMPLE 1

Each 1800 mg suppository contains:

| | |
|---|---|
| Calcitonin | 200 I.U. |
| Tromethamine citrate, monobasic | 4.00 mg |
| Methyl p-hydroxybenzoate | 0.40 mg |
| Propyl p-hydroxybenzoate | 0.04 mg |
| Mannitol | 36.90 mg |
| Albumin | 3.20 mg |
| Edetic acid, disodium calcium salt | 0.08 mg |
| Mass (Novata BD) | 1404.50 mg |
| Vaseline oil | 351.00 mg |

### EXAMPLE 2

| | |
|---|---|
| Calcitonin | 100 I.U. |
| Tromethamine tartrate, monobasic | 4.2 mg |
| Methyl p-hydroxybenzoate | 0.40 mg |
| Propyl p-hydroxybenzoate | 0.04 mg |
| Mannitol | 36.90 mg |
| Albumin | 3.20 mg |
| Mass (Novata BD) | 1404.30 mg |
| Vaseline oil | 351.00 mg |

### EXAMPLE 3

| | |
|---|---|
| Calcitonin | 200 I.U. |
| Tromethamine succinate, monobasic | 4.00 mg |
| Methyl p-hydroxybenzoate | 0.40 mg |
| Propyl p-hydroxybenzoate | 0.04 mg |
| Mannitol | 36.90 mg |
| Albumin | 3.20 mg |
| Mass (Novata BD) | 1404.50 mg |
| Vaseline oil | 351.00 mg |

### EXAMPLE 4

| | |
|---|---|
| Calcitonin | 200 I.U. |
| Tromethamine citrate, monobasic | 2.16 mg |
| Meglumine citrate, monobasic | 2.64 mg |
| Methyl p-hydroxybenzoate | 0.40 mg |
| Propyl p-hydroxybenzoate | 0.04 mg |
| Mannitol | 40.00 mg |
| Mass (Novata BD) | 1404.00 mg |
| Vaseline oil | 351.00 mg |

### EXAMPLE 5

| | |
|---|---|
| Calcitonin | 200 I.U. |
| Tromethamine citrate, monobasic | 2.16 mg |
| Meglumine citrate, monobasic | 2.64 mg |
| Methyl p-hydroxybenzoate | 0.40 mg |
| Propyl p-hydroxybenzoate | 0.04 mg |
| Mannitol | 36.80 mg |
| Albumin | 3.20 mg |
| Mass (Novata BD) | 1404.00 mg |
| Vaseline oil | 351.00 mg |

### EXAMPLE 6

| | |
|---|---|
| Calcitonin | 200 I.U. |
| Tromethamine citrate, monobasic | 2.16 mg |
| Meglumine citrate, monobasic | 2.64 mg |
| Methyl p-hydroxybenzoate | 0.40 mg |
| Propyl p-hydroxybenzoate | 0.04 mg |
| Glycine | 40.00 mg |
| Edetic acid, disodium calcium salt | 0.10 mg |
| Mass (Novata BD) | 1404.00 mg |
| Vaseline oil | 351.00 mg |

### EXAMPLE 7

| | |
|---|---|
| Calcitonin | 200 I.U. |
| Tromethamine citrate, monobasic | 2.16 mg |
| Meglumine citrate, monobasic | 2.64 mg |
| Methyl p-hydroxybenzoate | 0.40 mg |
| Propyl p-hydroxybenzoate | 0.04 mg |
| Glycine | 36.80 mg |
| Albumin | 3.20 mg |
| Mass (Novata BD) | 1404.00 mg |
| Vaseline oil | 351.00 mg |

The process for the preparation is hereinafter illustrated.
a) Preparation of the freeze-dried mixture.
   Calcitonin, buffer (i.e. tromethamine and meglumine salts), carrier (mannitol, glycine, etc.) and stabilizers (albumin) were dissolved in distilled water; a cold solution of p-hydroxybenzoates, which had previously been dissolved at boiling temperature, was added.

The water total amount to be used was determined so that the final solution contained 10% w/v of carrier, or carrier with albumin. 0.4 ml for each suppository were preferably used.

The pH of the solution was 3.8-4.3.

Whenever necessary, the solution was filtered through membrane, then the bulk freeze-drying process was carried out. Final vacuum was at least 15 »m Hg and the final (maximum) temperature of the product was 20°C.

Residual humidity must be lower than 0.3%. The product was sifted and calcitonin titre was determined by HPLC.
b) Preparation of the mass.
   The suppository mass was melted and vaseline oil was added. A clear liquid stable at 33-33.5°C was obtained.
c) Preparation of suppositories.
   About 1/10 of the mass, prepared as described in b), was added to the necessary amount of the freeze-dried product, said amount being determined according to the calcitonin titre. The resulting mixture was stirred with a fast mixer till a perfectly homogeneous suspension was obtained. The remaining of the melted mass was subsequently added. Mixing was continued till homogeneity, and the final product was partitioned in the appropriate PVC molds. The temperature throughout the process was 33-33.5°C.
Remark: Alternatively, the buffer, instead of being added to the calcitonin solution to be freeze-dried, can be dried, accurately milled and sifted, then mixed with the melted mass before casting.

In the above formulations, the tromethamine salt acts both as buffer and as absorption promoter.

In order to evaluate calcitonin bioavailability after rectal administration of the various formulations, the effect of the different buffers and components and the stability of the active ingredients, some pharmacological tests were prepared, according to the biological assay described in the Italian Official Pharmacopoeia (FUI).

The stability of the active ingredient during the preparation and partition steps was confirmed carrying out the biological assay on samples which had been kept at 33-33.5°C at least for 10 hours.

The obtained results led to the conclusion that calcitonin, formulated according to the present invention and rectally administered, is certainly absorbed. Absorption AUC relating to the different compositions are quantitatively comparable.

## Claims (Claims for the following Contracting State(s): AT, BE, CH, DE, FR, GB, IT, LI, LU, NL, SE, DK, MC)

1. Rectal pharmaceutical compositions, in the form of suppositories, containing calcitonin as the active ingredient, said calcitonin being freeze-dried on a proper support.

2. The compositions according to claim 1 further containing a pharmaceutically acceptable tromethamine or meglumine salt.

3. The compositions according to claim 2, characterized in that a tromethamine salt is used.

4. The compositions according to claim 3, characterized in that the tromethamine salt is selected from the group consisting of malonate, maleate, fumarate, tartrate, citrate, succinate, malate, glutamate, aspartate, glutarate, adipate, glucuronate.

5. The compositions according to claims 1-4 further containing bactericides selected from the group consisting of phenol compounds, in amounts ranging from 0.1 to 0.2% w/v; quaternary ammonium compounds, in amounts ranging from 0.005 to 0.03% w/v; mercurial compounds, in amounts ranging from 0.001 to 0.01% w/v.

6. The compositions according to the preceding claims characterized in that they contain edetic acid, or the salts thereof, as stabilizers in amounts ranging from 0.05 to 0.5 mg per suppository.

7. The compositions according to the preceding claims characterized in that calcium disodium edetate is the edetic acid salt.

8. A process for the preparation of rectal pharmaceutical compositions in the form of suppositories containing calcitonin, characterized in that calcitonin is freeze-dried on a suitable support and the resulting lyophilized calcitonin is added to the hydrophilic or lypophilic suppository masses, optionally containing other excipients.

## Claims (Claims for the following Contracting State(s): ES, GR)

1. A process for the preparation of rectal pharmaceutical compositions, in the form of suppositories, containing calcitonin as the active ingredient, characterized in that calcitonin is freeze-dried on a suitable support and the resulting lyophilized calcitonin is added to the hydrophilic or lypophilic suppository masses, optionally containing other excipients.

## Patentansprüche (Patentansprüche für folgende(n) Vertragsstaat(en): AT, BE, CH, DE, FR, GB, IT, LI, LU, NL, SE, DK, MC)

1. Arzneizusammensetzungen zur rektalen Verabreichung in der Form von Suppositorien, enthaltend Calcitonin als aktiver Bestandteil, wobei das Calcitonin auf einem geeigneten Träger gefriergetrocknet ist.

2. Zusammensetzungen nach Anspruch 1, welche weiterhin ein pharmazeutisch akzeptables Tromethamin- oder Megluminsalz enthalten.

3. Zusammensetzungen nach Anspruch 2, dadurch gekennzeichnet, daß ein Tromethaminsalz verwendet ist.

4. Zusammensetzungen nach Anspruch 3, dadurch gekennzeichnet, daß das Tromethaminsalz ausgewählt ist von der Gruppe, bestehend aus Malonat, Maleat, Fumarat, Tartrat, Citrat, Succinat, Malat, Glutamat, Aspartat, Glutarat, Adipat, Glukuronat.

5. Zusammensetzungen nach den Ansprüchen 1 bis 4, welche weiterhin Bakterizide enthalten, ausgewählt von der Gruppe, bestehend aus Phenolverbindungen, in Mengen, die von 0.1 bis 0.2 % w/v reichen; quarternären Ammoniumverbindungen, in Mengen, die von 0.005 bis 0.03 % w/v reichen; Quecksilberverbindungen, in Mengen, die von 0.001 bis 0.01 % w/v reichen.

6. Zusammensetzungen nach den vorhergehenden Ansprüchen, dadurch gekennzeichnet, daß sie Edetinsäure oder die Salze davon als Stabilisatoren in Mengen enthalten, die von 0.05 bis 0.5 mg je Suppositorium reichen.

7. Zusammensetzungen nach den vorhergehenden Ansprüchen, dadurch gekennzeichnet, daß Calciumdinatriumedetat das Edetinsäuresalz ist.

8. Verfahren zur Herstellung von Arzneimittelzusammensetzungen in der Form von Suppositorien, die Calcitonin enthalten, dadurch gekennzeichnet, daß das Calcitonin auf einem geeigneten Träger gefriergetrocknet wird und das erhaltene gefriergetrocknete Calcitonin den hydrophilen oder lypophilen Suppositorienmassen hinzugefügt wird, die wahlweise andere Arzneimittelträger enthalten.

## Patentansprüche (Patentansprüche für folgende(n) Vertragsstaat(en): ES, GR)

1. Verfahren zur Herstellung von Arzneimittelzusammensetzungen in der Form von Suppositorien, die Calcitonin enthalten, dadurch gekennzeichnet, daß das Calcitonin auf einem geeigneten Träger gefriergetrocknet wird und das erhaltene gefriergetrocknete Calcitonin den hydrophilen oder lypophilen Suppositorienmassen hinzugefügt wird, die wahlweise andere Arzneimittelträger enthalten.

## Revendications (Revendications pour l'(les) Etat(s) contractant(s) suivant(s): AT, BE, CH, DE, FR, GB, IT, LI, LU, NL, SE, DK, MC)

1. Compositions pharmaceutiques rectales, sous la forme de suppositoires, contenant de la calcitonine comme principe actif, ladite calcitonine étant lyophilisée sur un support adéquat.

2. Compositions selon la revendication 1 contenant de plus un sel de trométhamine ou de méglumine pharmaceutiquement acceptable.

3. Compositions selon la revendication 2, caractérisées par l'utilisation d'un sel de trométhamine.

4. Compositions selon la revendication 3, caractérisées par le choix du sel de trométhamine dans le groupe comprenant du malonate, maléate, fumarate, tartrate, citrate, succinate, malate, glutamate, aspartate, glutarate, adipate, glucuronate.

5. Compositions selon les revendications 1 à 4 contenant de plus des bactéricides choisis dans le groupe comprenant des composés phénoliques, dans des quantités allant de 0,1 à 0,2% en poids/volume, des composés ammonium quaternaire, dans des quantités allant de 0,005 à 0,03% en poids/volume; des composés de mercure, dans des quantités allant de 0,001 à 0,01% en poids/volume.

6. Compositions selon les précédentes revendications caractérisées en ce qu'elles contiennent de l'acide édetique, ou des sels de celui-ci, comme stabilisants dans des quantités allant de 0,05 à 0,5 mg par suppositoire.

7. Compositions selon les revendications précédentes caractérisées en ce que le sel de l'acide édetique est le disodium édetate de calcium.

8. Procédé pour la préparation de compositions pharmaceutiques rectales sous la forme de suppositoires contenant de la calcitonine, caractérisé en ce que la calcitonine est lyophilisée sur un support convenable et que la calcitonine lyophilisée résultante est ajoutée aux masses hydrophile ou lipophile du suppositoire, pouvant contenir en option d'autres excipients.

## Revendications (Revendications pour l'(les) Etat(s) contractant(s) suivant(s): ES, GR)

1. Procédé pour la préparation de compositions pharmaceutiques rectales, sous la forme de suppositoires, contenant de la calcitonine comme principe actif, caractérisé en ce que la calcitonine est lyophilisée sur un support convenable et que la calcitonine lyophilisée résultante est ajoutée aux masses hydrophile ou lipophile du suppositoire, pouvant contenir en option d'autres excipients.
